# EUROPÄISCHE PATENTSCHRIFT

(11) **EP 0 778 921 B1**
(45) Veröffentlichungstag und Bekanntmachung des Hinweises auf die Patenterteilung: **04.04.2001**
(21) Anmeldenummer: 96914066.4
(22) Anmeldetag: 10.05.1996
(51) Int. Cl.: F02M 25/08, F04B 45/047, F04B 49/24

(54) **PUMPVORRICHTUNG**
PUMPING DEVICE
DISPOSITIF DE POMPAGE

(30) Priorität: 30.06.1995 DE 19523935
(43) Veröffentlichungstag der Anmeldung: 18.06.1997
(73) Patentinhaber: ROBERT BOSCH GMBH, 70442 Stuttgart (DE)
(72) Erfinder: KRIMMER, Erwin, D-73655 Plüderhausen (DE); DENZ, Helmut, D-70176 Stuttgart (DE); SCHULZ, Wolfgang, D-74321 Bietigheim-Bissingen (DE); WILD, Ernst, D-71739 Oberriexingen (DE); KUHNT, Winfried, D-70193 Stuttgart (DE); SCHWEGLER, Helmut, D-74385 Pleidelsheim (DE); BLUMENSTOCK, Andreas, D-71638 Ludwigsburg (DE); MIEHLE, Tilman, D-71394 Kernen (DE); ZIMMERMANN, Manfred, D-74906 Bad Rappenau (DE)
(86) Internationale Anmeldenummer: DE9600815
(87) Internationale Veröffentlichungsnummer: WO9702421

(56) Entgegenhaltungen:
- WO-A-94/15090
- WO-A-94/27131

## Beschreibung

### Stand der Technik

Die Erfindung geht aus von einer Pumpvorrichtung, insbesondere für ein Brennstoffverdunstungs-Rückhaltesystem einer Brennkraftmaschine, nach der Gattung des Anspruchs 1. Es ist schon eine Pumpvorrichtung bekannt (WO 94/15090), die zur Dichtheitsprüfung eines Brennstoffverdunstungs-Rückhaltesystems vorgesehen ist, um mittels der Pumpvorrichtung ein definiertes Luftvolumen über einen Belüftungsanschluß eines Adsorptionsfilters einem Brennstofftank der Brennkraftmaschine zuzuführen, so daß eine Druckerhöhung bewirkt wird. Um festzustellen, ob das Brennstoffverdunstungs-Rückhaltesystem druckdicht ist, wird nach abgeschlossenem Druckaufbau geraume Zeit gewartet, um bei einem Druckabbau im Brennstoffverdunstungs-Rückhaltesystem auf eine Leckage zu schließen, wobei die zum Druckabbau verstrichene Zeit ein Maß für die Größe der Leckageöffnung ist. Weiterhin umfaßt das Brennstoffverdunstungs-Rückhaltesystem ein Regenerierventil, das zwischen dem Adsorptionsfilter und einem Ansaugrohr der Brennkraftmaschine vorgesehen ist, um mittels des Regenerierventils den im Adsorptionsfilter zwischengespeicherten Brennstoffdampf in das Ansaugrohr einzuleiten.

Die im Stand der Technik angegebene Pumpvorrichtung besitzt eine Pumpmembran, welche zum Antrieb wechselweise mit Unterdruck und mit Umgebungsdruck beaufschlagt wird. Der Unterdruck wird bei laufender Brennkraftmaschine vom Ansaugrohr der Brennkraftmaschine über einen Unterdruckschlauch entnommen und über ein Schaltventil, das beispielsweise in Form eines Elektromagnetventils ausgebildet ist, einem vom Schaltventil und der Pumpmembran begrenzten Pumpraum der Pumpvorrichtung zugeführt. Durch Schalten des Schaltventils wird wechselweise Unterdruck und Umgebungsdruck im Pumpraum eingestellt. Bei Beaufschlagung des Pumpraums mit Unterdruck bewegt sich die Pumpmembran entgegen der Druckkraft einer Pumpfeder, wobei Luft aus einer Zuführleitung in einen dem Pumpraum gegenüberliegenden Förderraum einströmt, der von der Pumpmembran und zwei Sperrventilen, einem Unterdruckventil und einem Überdruckventil, abgeschlossen ist. Bei der anschließenden Beaufschlagung des Pumpraums mit Umgebungsdruck bewegt sich die Pumpmembran unterstützt durch die Druckkraft der Pumpfeder in entgegengesetzter Richtung, wobei die im Förderraum eingeschlossene Luft verdichtet wird. Beim Erreichen eines bestimmten Überdrucks im Förderraum öffnet das Überdruckventil, so daß die im Förderraum verdichtete Umgebungsluft über die Förderleitung in den Belüftungsanschluß des Adsorptionsfilters strömt, um eine Druckerhöhung im Brennstofftank zu bewirken.

Der Umschaltvorgang mittels des Schaltventils wird im angegebenen Stand der Technik von einem dem Fachmann bekannten, sogenannten Reed-Schalter gesteuert. Der Reed-Schalter wird von magnetischen Kräften betätigt und ist zum Beispiel an einer Außenfläche einer Hülse angebracht, in der ein an der Pumpmembran angebrachter Pumpstößel längsverschiebbar geführt ist. An einer dem Reed-Schalter gegenüberliegenden Seite der Hülse ist ein Dauermagnet vorgesehen, um abhängig von der Lage des Pumpstößels in der Hülse eine entsprechende Änderung des magnetischen Feldes des Dauermagneten am Reed-Schalter zu bewirken, so daß beim Erreichen der Endlage des Pumpstößels der Reed-Schalter entsprechend betätigt wird. Die Lagebestimmung des Pumpstößels mittels des Reed-Schalters ist jedoch mit hohen Toleranzen verbunden und erlaubt daher keine präzise Bestimmung der Lage des Pumpstößels in der Hülse beziehungsweise der Lage der Pumpmembran, so daß nur relativ ungenau die Bestimmung der Größe der Leckageöffnung möglich ist.

Eine von im Ansaugrohr herrschenden Unterdruck angetriebene Pumpvorrichtung ist üblicherweise in der Nähe des Adsorptionsfilters vorgesehen, der sich vorzugsweise im Bereich des Brennstofftanks eines Kraftfahrzeugs befindet. Der Brennstofftank ist gewöhnlich im Heckbereich des Kraftfahrzeugs untergebracht, so daß sich ein relativ langer Unterdruckschlauch von der Pumpvorrichtung am Adsorptionsfilter zum Ansaugrohr der Brennkraftmaschine ergibt. Da eine Leckage oder ein Abreißen des Unterdruckschlauchs die Pumpvorrichtung außer Betrieb setzen würde, muß ein derartiger Unterdruckschlauch in besonders geschützter Weise am Fahrzeugboden des Kraftfahrzeugs verlegt werden, was jedoch erhebliche Kosten verursacht.

Die vom Unterdruck des Ansaugrohres angetriebene Pumpvorrichtung besitzt weiterhin den Nachteil, daß eine Dichtheitsdiagnose nur bei ausreichendem Unterdruck im Ansaugrohr durchführbar ist. Die Dichtheitsdiagnose ist daher im wesentlichen auf den durch einen hohen Unterdruck gekénnzeichneten Leerlaufbereich der Brennkraftmaschine beschränkt. Im oberen Teillastbereich oder im Vollastbereich sowie bei abgeschalteter Brennkraftmaschine ist kein ausreichender Unterdruck zum Antrieb der Pumpvorrichtung vorhanden, so daß keine Dichtheitsdiagnose möglich ist.

Während der Dichtheitsdiagnose wird darüber hinaus bei jedem Schaltvorgang des Schaltventils beim Übergang vom Umgebungsdruck zum Unterdruck Luft aus dem Pumpraum in das Ansaugrohr abgesaugt. Dies kann jedoch zu Schwankungen der im Ansaugrohr strömenden Luftmenge führen, welche bereits von einem stromaufwärts der Entnahmestelle des Unterdruckanschlusses im Ansaugrohr gelegenen Drosselorgan zugemessen ist, so daß sich insbesondere im kritischen Bereich des Leerlaufs der Brennkraftmaschine durch die pulsierende Einleitung der Luft Schwierigkeiten beim Aufbereiten eines genau einzuhaltenden Brennstoff-Luft-Verhältnisses in den Brennräumen der Brennkraftmaschine ergeben können.

### Vorteile der Erfindung

Die erfindungsgemäße Pumpvorrichtung mit den kennzeichnenden Merkmalen des Anspruchs 1 hat demgegenüber den Vorteil, daß es auf einfache Art und Weise mit der erfindungsgemäßen Pumpvorrichtung zu jeder Zeit möglich ist, unabhängig von den Betriebsbereichen der Brennkraftmaschine und selbst bei abgestellter Brennkraftmaschine, eine Dichtheitsdiagnose durchzuführen. Vorteilhafterweise kann der im Stand der Technik benötigte Unterdruckanschluß an der Pumpvorrichtung entfallen, wodurch die erfindungsgemäße Pumpvorrichtung ausfallsicherer und kostengünstiger ist. Besonders vorteilhaft ist, daß mit der erfindungsgemäßen Pumpvorrichtung selbst kleinste Leckageöffnungen von weniger als einem Millimeter Durchmesser feststellbar sind. Vorteilhafterweise kann die Dichtheitsdiagnose mittels der erfindungsgemäßen Pumpvorrichtung ohne eine Luftzufuhr in das Ansaugrohr bewerkstelligt werden, so daß eine nachteilige Beeinflussung der Gemischaufbereitung ausgeschlossen ist.

Durch die in den Unteransprüchen aufgeführten Maßnahmen sind vorteilhafte Weiterbildungen und Verbesserungen der im Anspruch 1 angegebenen Pumpvorrichtung möglich. Die erfindungsgemäße Pumpvorrichtung zeichnet sich weiterhin durch eine besonders kompakte Bauweise aus, bei der die elektrischen Komponenten in geschützter Weise im Gehäuse untergebracht sind, so daß bei einem Eindringen von Wasser oder von Brennstoff in die Pumpvorrichtung kein Ausfall der Pumpvorrichtung zu befürchten ist. Darüber hinaus erlaubt die vorgesehene, hohe Pumpfrequenz der Pumpvorrichtung in Verbindung mit einer besonders genauen Lagebestimmung der Pumpmembran mittels einer am Pumpstößel angebrachten Kontaktscheibe und vorgesehenen Kontaktfahnen eine präzise Bestimmung kleinster Leckageöffnungen, wobei das Betriebsgeräusch der Pumpvorrichtung nur gering ist. Besonders vorteilhaft ist, daß wesentliche Ansteuerungsfunktionen der Pumpvorrichtung mittels einer im Gehäuse mit elektrischen Bauteilen bestückten Kontaktplatte selbsttätig vorgenommen werden können, so daß unter anderem nur wenige Verbindungsleitungen zu einem externen elektronischen Steuergerät erforderlich sind. Außerdem ist mittels der erfindungsgemäßen Pumpvorrichtung auch die Erkennung großer Leckageöffnungen des Brennstoffverdunstungs-Rückhaltesystems möglich, die beispielsweise auf einen fehlenden Tankdeckel an einem Brennstofftank der Brennkraftmaschine zurückzuführen sind.

### Zeichnung

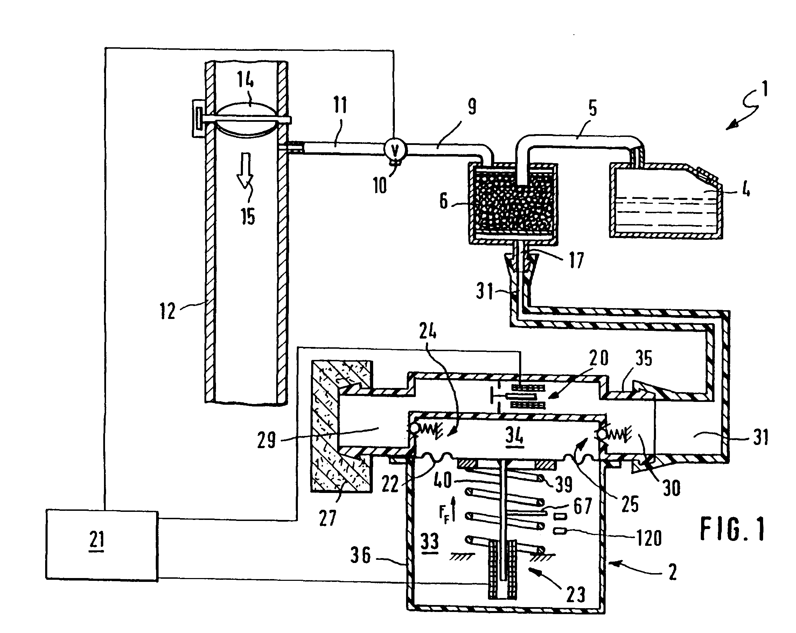

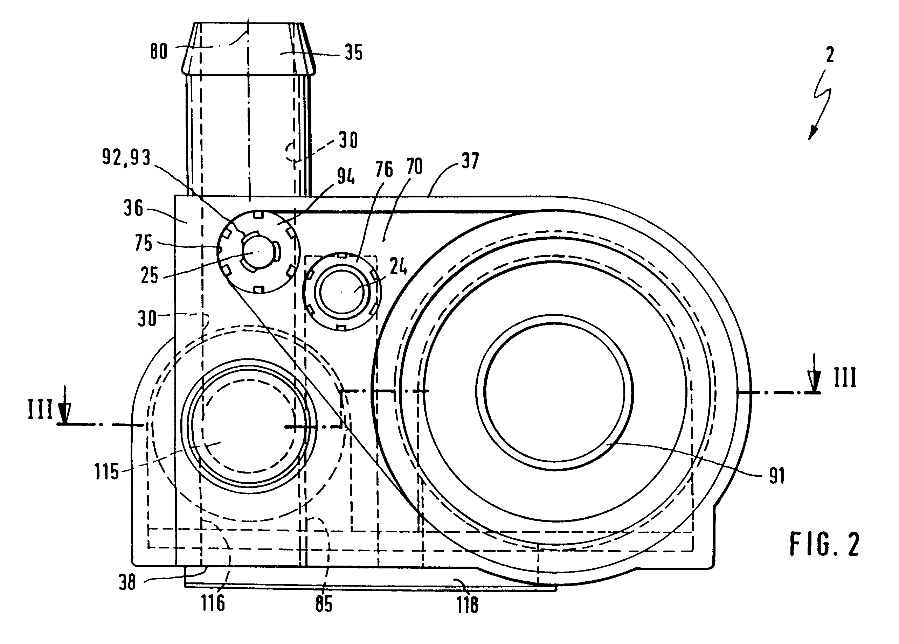

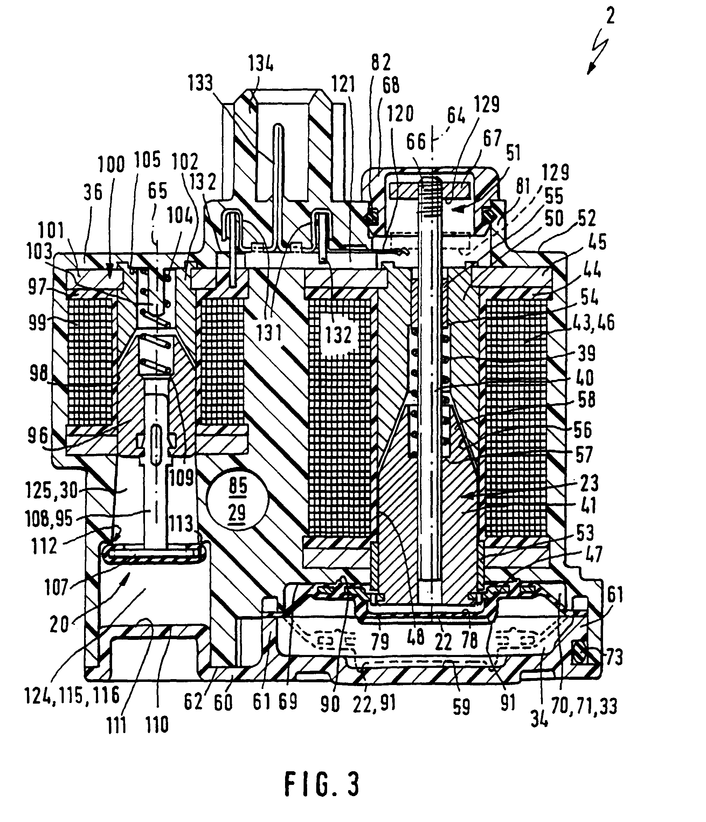

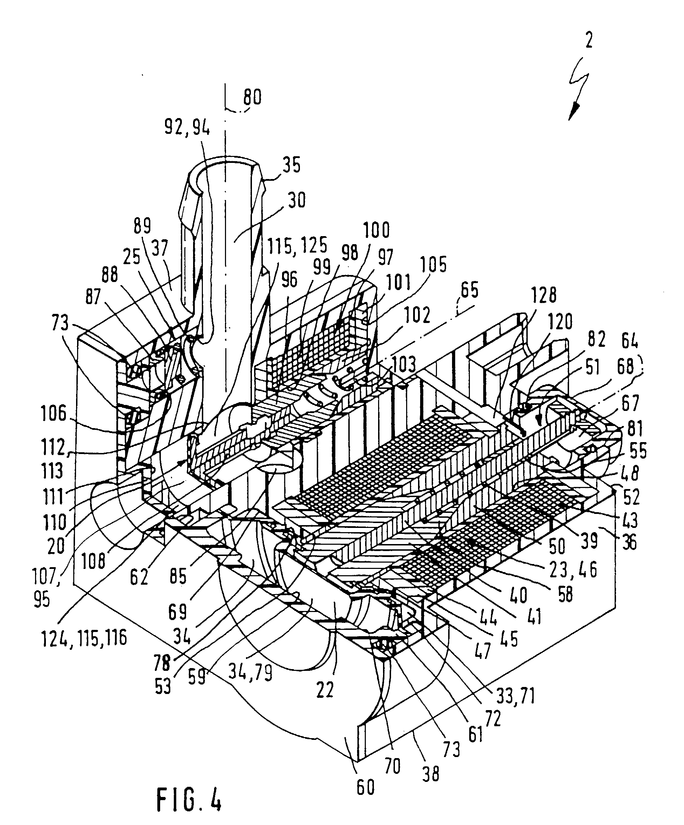

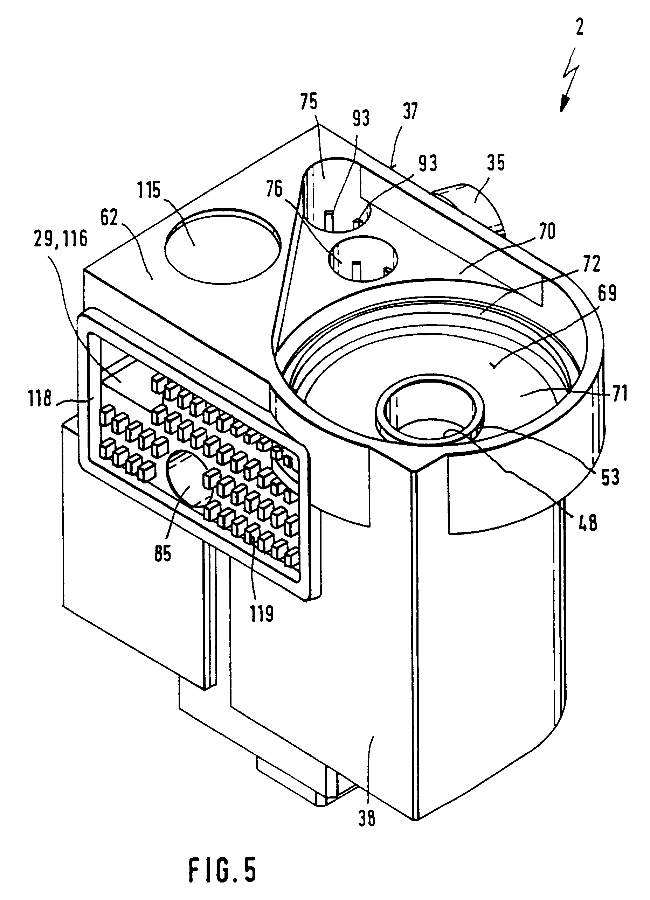

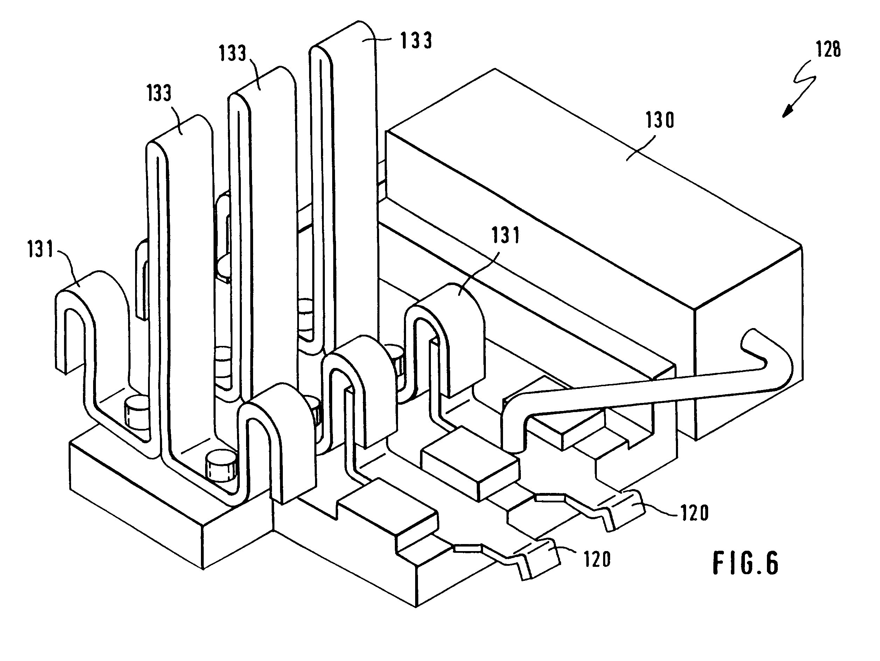

Ein Ausführungsbeispiel der Erfindung ist in der Zeichnung vereinfacht dargestellt und in der nachfolgenden Beschreibung näher erläutert. Es zeigen Figur 1 ein Brennstoffverdunstungs-Rückhaltesystem für eine Brennkraftmaschine, das eine in schematisch vereinfachter Funktionsweise dargestellte erfindungsgemäße Pumpvorrichtung besitzt, Figur 2 eine Seitenansicht der erfindungsgemäßen Pumpvorrichtung, Figur 3 einen Schnitt entlang einer Linie III-III in Figur 2, Figur 4 eine in teilweiser aufgeschnittener Ansicht perspektivisch dargestellte erfindungsgemäße Pumpvorrichtung, Figur 5 eine perspektivische Rückansicht der erfindungsgemäßen Pumpvorrichtung, Figur 6 eine perspektivische Ansicht eines Bauteils der erfindungsgemäßen Pumpvorrichtung.

### Beschreibung des Ausführungsbeispiels

Die Figur 1 zeigt ein mit 1 gekennzeichnetes Brennstoffverdunstungs-Rückhaltesystem für eine nicht näher dargestellte Brennkraftmaschine, das mit einer in schematisch vereinfachter Funktionsweise dargestellten erfindungsgemäßen Pumpvorrichtung 2 ausgestattet ist, die zu Diagnosezwecken einen Überdruck im Brennstoffverdunstungs-Rückhaltesystem 1 aufbaut. Das Brennstoffverdunstungs-Rückhaltesystem 1 umfaßt weiterhin einen Brennstofftank 4 zur Versorgung der Brennkraftmaschine mit Brennstoff und einen über eine Tankleitung 5 mit dem Brennstofftank 4 verbundenen Adsorptionsfilter 6. Der Adsorptionsfilter 6 ist mit einem Adsorptionsmedium, insbesondere mit Aktivkohle, gefüllt und über eine Verbindungsleitung 9 mit einem Regenerierventil 10 verbunden, das über eine Ventilleitung 11 an ein Ansaugrohr 12 der Brennkraftmaschine angeschlossen ist. Die Ventilleitung 11 mündet beispielsweise stromabwärts einer drehbar in das Ansaugrohr 12 der Brennkraftmaschine eingebrachten Drosselklappe 14, in welchem ein Luft- oder ein Brennstoff-Luft-Gemisch in Richtung eines eingezeichneten Pfeils 15 strömt. Beim Betrieb der Brennkraftmaschine herrscht Unterdruck im Ansaugrohr 12, mit dessen Hilfe bei geöffnetem Regenerierventil 10 die Brennstoffdämpfe aus dem Brennstofftank 4 abgesaugt werden. Die Brennstoffdämpfe gelangen dabei vom Brennstofftank 4 über die Tankleitung 5 in den Adsorptionsfilter 6 und von diesem in die Verbindungsleitung 9, wobei Umgebungsluft durch den Unterdruck im Ansaugrohr 12 über einen am Adsorptionsfilter 6 vorgesehenen Belüftungsanschluß 17 angesaugt wird, so daß der im Adsorptionsfilter 6 zwischengespeicherte Brennstoff mitgerissen wird. Die im Adsorptionsfilter 6 zwischengespeicherten Brennstoffdämpfe vermischen sich mit der über den Belüftungsanschluß 17 einströmenden Umgebungsluft. Über das zum Beispiel elektromagnetisch betätigbar ausgebildete und von einem elektronischen Steuergerät 21 getaktet angesteuerte Regenerierventil 10 gelangen die Brennstoffdämpfe über das Regenerierventil 10 und die Ventilleitung 11 in das Ansaugrohr 12, um anschließend in wenigstens einem Brennraum der Brennkraftmaschine zu verbrennen.

Zur Dichtheitsprüfung des Brennstoffverdunstungs-Rückhaltesystems 1 wird das Regenerierventil 10 geschlossen. Anschließend wird dem Brennstofftank 4 mittels der Pumpvorrichtung 2 über den Adsorptionsfilter 6 ein definiertes Luftvolumen zugeführt, um eine Druckerhöhung zu bewirken. Nach abgeschlossenem Druckaufbau wird geraume Zeit gewartet, bis sich der Druck gegebenenfalls aufgrund einer Leckage im Brennstoffverdunstungs-Rückhaltesystem 1 wieder abgebaut hat, wobei die zum Druckabbau verstrichene Zeit ein Maß für die Größe der im Brennstoffverdunstungs-Rückhaltesystem 1 aufgetretenen Leckageöffnung ist. Diese auch als Überdruckmethode bekannte Dichtheitsprüfung des Brennstoffverdunstungs-Rückhaltesystems 1 ermöglicht, Leckageöffnungen in einer Größenordnung von weniger als einem Millimeter Durchmesser festzustellen. Wird der Überdruck im Brennstoffverdunstungs-Rückhaltesystem 1 selbst nach einer bestimmten Anzahl von Pumphüben der Pumpmembran 22 nicht erreicht, so kann auf ein Grobleck oder auf einen fehlenden Tankdeckel am Brennstofftank 4 geschlossen werden. In diesem Fall ist es möglich, über das mit der Pumpvorrichtung 2 verbundene elektronische Steuergerät 21 eine zum Beispiel im Fahrzeuginnern untergebrachte Anzeigevorrichtung anzusteuern, um so den Fahrer über die aufgetretene Fehlfunktion des Brennstoffverdunstungs-Rückhaltesystems 1 entsprechend zu informieren.

Der zu Überprüfungszwecken benötigte Überdruck wird von der erfindungsgemäßen Pumpvorrichtung 2 bereitgestellt, die beim Pumpvorgang Umgebungsluft, beispielsweise über einen in oder an einem Gehäuse 36 der Pumpvorrichtung 2 angeordneten Umgebungsluftfilter 27, in eine Zuführleitung 29 ansaugt, um diese danach mit erhöhtem Druck in eine Förderleitung 30 zu pumpen. Die Förderleitung 30 ist beispielsweise über eine separate Leitung 31 an den Belüftungsanschluß 17 des Adsorptionsfilters 6 angeschlossen. Die Pumpvorrichtung 2 setzt sich aus mehreren, funktionell voneinander getrennten Einzelkomponenten zusammen, die im Gehäuse 36 untergebracht sind und im wesentlichen ein elektromagnetisch betätigbares Absperrventil 20 und einen Pumpteil 23 umfassen. Der Pumpteil 23 ist zum Verdichten der Umgebungsluft vorgesehen und setzt sich aus einer Pumpmembran 22, einem Pumpstößel 40, einer die Lage des Pumpstößels 40 erfassenden Einrichtung 67, 120, einer Pumpfeder 39 und einer von einem Unterdruckventil 24 und einem Überdruckventil 25 gebildeten Ventilanordnung zusammen. Die Pumpmembran 22 unterteilt den Pumpteil 23 in einen in Figur 1 unterhalb der Pumpmembran 22 dargestellten Pumpraum 33 und in einen oberhalb der Pumpmembran 22 dargestellten Förderraum 34. Der Förderraum 34 wird von der Pumpmembran 22, dem Unterdruckventil 24 und dem Überdruckventil 25 druckdicht von der Umgebung abgeschlossen.

Während des Betriebs der Pumpvorrichtung 2 nimmt das zwischen der Zuführleitung 29 und der Förderleitung 30 parallel zu den Ventilen 24, 25 geschaltete Absperrventil 20 eine Schließstellung ein, um eine Strömungsverbindung der Zuführleitung 29 zu der Förderleitung 30 zu unterbrechen. Ist kein Betrieb der Pumpvorrichtung 2 beziehungsweise keine Dichtheitsprüfung des Brennstoffverdunstungs-Rückhaltesystems 1 erwünscht, so verbleibt das Absperrventil 20 in der in Figur 1 dargestellten Offenstellung. In Offenstellung des Absperrventils 20 kann bei offenem Regenerierventil 10 zur Regeneration des Adsorptionsfilters 6 Umgebungsluft über den an der Zuführleitung 29 vorgesehenen Umgebungsluftfilter 27 in die Förderleitung 30 und von dieser über die Leitung 31 und den Belüftungsanschluß 17 in den Adsorptionsfilter 6 einströmen.

Wie in der Figur 2, einer Seitenansicht der Pumpvorrichtung 2, dargestellt ist, besitzt die Pumpvorrichtung 2 ein im wesentlichen als Block ausgebildetes, massives, kompaktes, einteiliges, quaderförmiges Gehäuse 36, mit einem aus einer oberen Begrenzungsfläche 37 herausragenden stutzenförmigen Anschluß 35. Der Anschluß 35 bildet die in Figur 1 dargestellte Förderleitung 30 und dient zum Anschluß der Pumpvorrichtung 2, zum Beispiel über eine auf den Anschluß 35 aufgeschobenen Leitung 31 oder ohne Leitung 31 direkt an den Belüftungsanschluß 17 des Adsorptionsfilters 6. Das Gehäuse 36 der Pumpvorrichtung 2 ist aus Kunststoff, beispielsweise in Kunststoffspritzgußtechnik hergestellt, mit den erforderlichen Öffnungen, Erhebungen und Ausbuchtungen.

Wie in der Figur 3, einem Schnitt entlang einer Linie III-III in Figur 2, dargestellt ist, sind im Gehäuse 36 der Pumpteil 23 und das Absperrventil 20 untergebracht. Erfindungsgemäß ist der Pumpteil 23 derart ausgebildet, daß dieser in Form eines elektromagnetischen Antriebs die Pumpmembran 22 antreibt, wobei auch das Absperrventil 20 elektromagnetisch betätigbar ausgebildet ist. Der Pumpteil 23 besitzt hierzu einen am Pumpstößel 40 angebrachten magnetischen Anker 41, der von magnetischen Kräften eines in einem zylindrischen Spulengehäuse 44 vorgesehenen und von einer Erregerspule 43 gebildeten Elektromagneten 46 bewegbar ist, um so die Pumpmembran 22 elektromagnetisch, vorzugsweise mit einer relativ hohen Pumpfrequenz, anzutreiben. Das Spulengehäuse 44 besteht vorzugsweise aus Kunststoff. Die Erregerspule 43 ist im zylindrischen Spulengehäuse 44 einliegend untergebracht und mit dem Spulengehäuse 44 an einem bügelförmigen Magnetgehäuseträger 45 befestigt. Die Pumpmembran 22 ist zum Beispiel über einen Tellerteil 47 mit dem Anker 41 fest verbunden. Der Tellerteil 47 ist vorgesehen, um der Pumpmembran 22 eine gewisse Steifigkeit zu verleihen. Die Pumpmembran 22 ist beispielsweise als Spritzteil aus Kunststoff hergestellt und zum Beispiel mittels Fluorsilikongewebe zusätzlich verstärkt.

Der Pumpstößel 40 ist mit seinem Anker 41 in einer im Spulengehäuse 44 vorgesehenen, von der Erregerspule 43 umgebenen zylindrischen Durchgangsöffnung 48 längsverschiebbar untergebracht, die sich entlang einer Längsachse 64 des Pumpstößels 40 erstreckt. Zur Lagerung des Ankers 41 ist eine gehäusefest am Spulengehäuse 44 und am Magnetgehäuseträger 45 angebrachte erste Lagerhülse 53 vorgesehen. Zur Lagerung des mit dem Anker 41 verbundenen Pumpstößels 40 ist weiterhin ein in der Durchgangsöffnung 48 im Spulengehäuse 44 untergebrachter Kern 50 vorgesehen, in welchem eine zweite Lagerhülse 55 untergebracht ist. Der Pumpstößel 40 ragt dabei mit einem Kontaktende 51 aus dem Magnetgehäuseträger 45 über eine Seitenfläche 52 des Gehäuse 36 hinaus und ist von einer hülsenförmigen Anformung 81 des Gehäuses 36 umgeben. Der Pumpstößel 40 besitzt an seinem Kontaktende 51 eine die Lage des Pumpstößels 40 erfassende Einrichtung, die zum Beispiel von einer auf ein Außengewinde 66 am Kontaktende 51 aufschraubaren Kontaktscheibe 67 und von Kontaktfahnen 120 gebildet wird, die von der Kontaktscheibe 67 elektrisch verbindbar sind. Ein in die hülsenförmige Anformung 81 einsetzbarer Verschlußdeckel 68 dichtet beispielsweise über einen Dichtring 82 das in der Anformung 81 liegende Kontaktende 51 des Pumpstößels 40 von der Umgebung ab.

Der im Spulengehäuse 44 untergebrachte Kern 50 besitzt eine sich zur Pumpmembran 22 hin konisch erweiternde Anschlagausnehmung 56, in welche der Anker 41 teilweise eintauchen kann. Ein der Pumpmembran 22 abgewandter Endbereich des Ankers 41 hat hierzu ein zum Pumpstößel 40 hin konisch zulaufend ausgebildetes Ende 58, um an entsprechend konisch zulaufenden Innenflächen der Anschlagausnehmung 56 des Kerns 50 anliegen zu können. Die Pumpfeder 39 ist um den Pumpstößel 40 herum vorgesehen und im Innern des Ankers 41 und des Kerns 50 untergebracht. Die Pumpfeder 39 stützt sich einerseits an einem im Innern des Kerns 50 vorgesehenen, von der zweiten Lagerhülse 55 gebildeten Kernanschlag 54 und andererseits an einer im Innern des Ankers 41 vorgesehenen Anlageschulter 57 ab. Im stromlosen Zustand der Erregerspule 43 wird die Pumpmembran 22 durch die Federkraft der Pumpfeder 39 an eine Innenwand 59 eines Gehäusedeckels 60 des Gehäuses 36 der Pumpvorrichtung 2 gedrückt und nimmt dabei die in Figur 3 gestrichelt dargestellte Lage an. Im bestromten Zustand der Erregerspule 43 liegt die Pumpmembran 22 an einer Innenfläche 69 einer die Pumpmembran 22 begrenzenden Pumpausnehmung 70 an und nimmt dabei die in Figur 3 durchgezogen dargestellte Lage ein.

Die Ausgestaltung des Ankers 41 und des Kerns 50 sowie die Dimensionierung der Pumpfeder 39 ist derart, daß bei an der Innenfläche 69 der Pumpausnehmung 70 anliegender Pumpmembran 22 stets ein geringer axialer Abstand des Ankers 41 von den konisch zulaufenden Innenflächen der Anschlagausnehmung 56 des Kerns 50 vorhanden ist, um beim Betrieb ein Anliegen des Ankers 41 an den Innenflächen der Anschlagausnehmung 56 weitestgehend zu vermeiden, wodurch das Betriebsgeräusch der Pumpvorrichtung 2 verringert wird. Die Pumpmembran 22 besitzt eine tellerförmige Form und hat an einer der Innenfläche 69 der Pumpausnehmung 70 zugewandten Stirnfläche 78 und an einer dem Gehäusedeckel 60 zugewandten Stirnfläche 79 zumindest eine konzentrisch um die Längsachse 64 umlaufende Anlagewulst 90 beziehungsweise eine Anlagewulst 91. Beim Anliegen der Pumpmembran 22 an der Innenfläche 69 der Pumpausnehmung 70 oder an der Innenwand 59 des Gehäusedeckels 60 verformt sich die Anlagewulst 90 beziehungsweise die Anlagewulst 91 elastisch, wodurch eine weitere Verringerung des Betriebsgeräuschs der Pumpvorrichtung 2 möglich ist.

Wie in der Figur 5, einer perspektivischen Rückansicht der Pumpvorrichtung 2 ohne Pumpmembran 22 und ohne Gehäusedeckel 60 dargestellt ist, weist die Pumpausnehmung 70 eine tropfenförmige Außenkontur auf, welche die Pumpmembran 22 und Ventilöffnungen 75, 76 der Ventile 24, 25 mit einschließt. Die Pumpausnehmung 70 ist aus einer Stirnfläche 62 des Gehäuses 36 ausgenommen und im vorgesehenen Bereich der Pumpmembran 22 zum Spulengehäuse 44 bis zur Innenfläche 69 hin vertiefter ausgebildet als der die Ventilöffnungen 75, 76 umgebende Bereich, um die Pumpmembran 22 in einer der Form der Pumpmembran 22 entsprechenden, beispielsweise zylindrischen Teilausnehmung 71 in der Pumpausnehmung 70 aufzunehmen. Die Teilausnehmung 71 ist über einen kreisförmigen Rand 72 von der Pumpausnehmung 70 abgegrenzt, um die etwas über den Rand 72 überstehende Pumpmembran 22 zwischen dem Rand 72 und einem am Gehäusedeckel 60 von der Innenwand 59 abstehenden, in den Rand 72 teilweise eingreifenden Steg 61 einzuspannen. Der Steg 61 ist in Figur 3 und in Figur 4 dargestellt, welche die Pumpvorrichtung 2 in teilweise aufgeschnittener Ansicht zeigt. Der vorzugsweise aus Kunststoff bestehende Gehäusedeckel 60 überdeckt dabei die Stirnfläche 62 des Gehäuses 36 und ist zum Beispiel durch Klemmung oder Verrasten des Stegs 61 in der Teilausnehmung 71 gehalten. Es ist jedoch auch möglich, den Gehäusedeckel 60 auf andere Art und Weise zu befestigen, beispielsweise durch Ankleben an die Stirnfläche 62, durch Ultraschallverschweißen mit dem Gehäuse 36 oder dergleichen. Ein zum Beispiel in Form eines O-Rings ausgebildeter Dichtring 73 am Gehäusedeckel 60 schließt die Pumpausnehmung 70 druckdicht von der Umgebung ab. Die in der Teilausnehmung 71 untergebrachte Pumpmembran 22 unterteilt den zwischen der Teilausnehmung 71 und dem Gehäusedeckel 60 abgeschlossenen Raum in zwei druckdicht voneinander getrennte Räume 33, 34, wobei der Raum 33 den Pumpraum 33 und der Raum 34 den Förderraum 34 in Figur 1 darstellt. Der Pumpraum 33 wird von der Pumpmembran 22 und der Innenfläche 69 der Pumpausnehmung 70 abgeschlossen. Der Förderraum 34 wird von der Pumpmembran 22, der Wandung der Pumpausnehmung 70 und der Innenwand 59 des Gehäusedeckels 60 abgeschlossen und erstreckt sich bis zu den Ventilen 24, 25.

Der Förderraum 34 ist über das in Figur 2 links dargestellte Überdruckventil 25 mit der Förderleitung 30 verbunden, die sich entlang einer quer zur Längsachse 64 erstreckenden, in Figur 4 eingezeichneten Stutzenlängsachse 80 des Anschlusses 35 im Gehäuse 36 erstreckt. Der Pumpraum 33 ist über eine nicht näher dargestellte, im Gehäuse 36 vorgesehene Entlüftungsverbindung mit der Umgebung verbunden beziehungsweise mit Umgebungsdruck beaufschlagt. Das Unterdruckventil 24 ist über einen parallel versetzt zur Stutzenlängsachse 80 im Gehäuse 36 vorgesehenen Ansaugkanal 85 mit der Umgebung verbunden. Der Ansaugkanal 85 erstreckt sich von der oberen Begrenzungsfläche 37 zu einer der oberen Begrenzungsfläche 37 gegenüberliegenden unteren Begrenzungsfläche 38 des Gehäuses 36.

Das in Figur 4 dargestellte Überdruckventil 25 setzt sich im wesentlichen aus einer in die Ventilöffnung 75 eingebrachten, abgestuft ausgebildeten Ventilhülse 87, einem aus elastischem Material bestehenden scheibenförmigen Ventilschließkörper 88, einer Ventilfeder 89 und einer Ventilfederaufnahme 92 zusammen. Die Ventilfederaufnahme 92 wird zum Beispiel von in die Ventilöffnung 75 hineinragenden, in Figur 2 und Figur 5 sichtbaren Stiften 93 gebildet, die im Mündungsbereich zwischen der Ventilöffnung 75 und der Förderleitung 30 einen ringförmigen Absatz 94 bilden, an dem sich die um die Stifte 93 herum vorgesehene Ventilfeder 89 mit ihrem einen Ende abstützen kann. Mit ihrem anderen Ende stützt sich die Ventilfeder 89 an dem scheibenförmigen Ventilschließkörper 88 ab, der durch die Federkraft der Ventilfeder 89 gegen einen ringförmigen Dichtsitz 106 der Ventilhülse 87 angedrückt wird, um die Ventilöffnung 75 abzudichten, so daß eine Strömungsverbindung vom Förderraum 34 zur Förderleitung 30 unterbrochen ist. Erst bei von der Dimensionierung der Ventilfeder 89 abhängigem Überdruck im Förderraum 34 hebt der Ventilschließkörper 88 von dem Dichtsitz 106 an der Ventilhülse 87 ab, um die im Förderraum 34 von der Pumpmembran 22 verdichtete Luft über die vom Überdruckventil 25 geöffnete Ventilöffnung 75 bei geschlossenem Unterdruckventil 24 in die Förderleitung 30 abzugeben. Der Aufbau des Unterdruckventils 24 erfolgt in entsprechender Weise dem des Überdruckventils 25, mit dem Unterschied, daß der Ventilschließkörper eine gegenüber dem Überdruckventil 25 umgekehrte Ventilschließrichtung aufweist, um bei von der Dimensionierung der Ventilfeder abhängigem, bestimmtem Unterdruck im Förderraum 34 von seinem Dichtsitz abzuheben, so daß Luft über die vom Unterdruckventil 24 geöffnete Ventilöffnung 76 aus dem Ansaugkanal 85 in den Förderraum 34 angesaugt werden kann.

Im Innern des Gehäuses 36 der Pumpvorrichtung 2 ist weiterhin das Absperrventil 20 untergebracht. Das Absperrventil 20 besitzt ein Ventilschließglied 95, das seitlich versetzt zum Pumpteil 23 mit einer in etwa parallel zur Längsachse 64 des Pumpstößels 40 verlaufenden Ventilachse 65 im Gehäuse 36 angeordnet ist, so daß zwei, voneinander unabhängige Bewegungsachsen 64, 65 vorhanden sind. Das Absperrventil 20 ist erfindungsgemäß elektromagnetisch betätigbar ausgebildet und besitzt hierzu einen am Ventilschließglied 95 angebrachten magnetischen Anker 96, der von magnetischen Kräften eines in einem zylindrischen Spulengehäuse 97 des Absperrventils 20 vorgesehenen und von einer Erregerspule 99 gebildeten Elektromagneten 100 bewegbar ist. Die Erregerspule 99 ist im zylindrischen Spulengehäuse 97 einliegend untergebracht und mit dem Spulengehäuse 97 an einem bügelförmigen Magnetgehäuseträger 101 befestigt. Das Spulengehäuse 97 besteht vorzugsweise aus Kunststoff. Der Anker 96 ist in einer im Spulengehäuse 97 vorgesehenen, von der Erregerspule 99 umgebenen zylindrischen Öffnung 98 untergebracht, die sich entlang der Ventilachse 65 des Absperrventils 20 erstreckt. In der Öffnung 98 ist ein hülsenförmiger Magnetkern 102 eingebracht, der gehäusefest am Magnetgehäuseträger 101 des Absperrventils 20 befestigt ist. In den hülsenförmigen Magnetkern 102 ragt ein von einer Innenwandung 105 im Gehäuse 36 abstehender Stift 103, um welchen herum eine Feder 104 angeordnet ist, die sich einerseits an der Innenwandung 105 des Gehäuses 36 und andererseits an einem im Innern des Ankers 96 vorgesehenen Anschlag 109 abstützt. Der Magnetgehäuseträger 101 des Absperrventils 20 und der Magnetgehäuseträger 45 des Pumpteils 23 sind beispielsweise einteilig ausgebildet und in den Kunststoff des Gehäuses 36 eingebettet.

Das Ventilschließglied 95 des Absperrventils 20 wird von einem tellerförmigen Teil 107 gebildet, der zum Beispiel einteilig an einem Stößel 108 angeformt ist. Der Stößel 108 ist zum Beispiel im Innern des magnetischen Ankers 96 befestigt. Wie in der Figur 3 dargestellt ist, ist der tellerförmige Teil 107 noch von einem elastischen Material, zum Beispiel Silikon, überzogen und in einem Querkanal 115 längsverschiebbar entlang der Ventilachse 65 untergebracht. Der Querkanal 115 erstreckt sich innerhalb des Gehäuses 36 von der Stirnfläche 62 in Richtung der Ventilachse 65 zum Spulengehäuse 97 des Absperrventils 20 hin. Der Querkanal 115 setzt sich aus einem einen zylindrischen Querschnitt aufweisenden Teil 124 und aus einem einen zum Spulengehäuse 97 hin konisch zulaufenden Querschnitt aufweisenden Teil 125 zusammen. Im zylindrischen Teil 124 des Querkanals 115 ist der tellerförmige Teil 107 des Ventilschließgliedes 95 untergebracht und in diesem entlang der Ventilachse 65 längsverschiebbar geführt. Wie in der Figur 2 und der Figur 4 dargestellt ist, mündet in den zylindrischen Teil 124 des Querkanals 115 ein beispielsweise einen rechteckförmigen Querschnitt aufweisender Strömungskanal 116. Der Strömungskanal 116 erstreckt sich innerhalb des Gehäuses 36 vom zylindrischen Teil 124 des Querkanals 115 parallel versetzt zur Stutzenlängsachse 80 des stutzenförmigen Anschlusses 35 und dem Ansaugkanal 85 und endet an der unteren Begrenzungsfläche 38 des Gehäuses 36. Der konische Teil 125 des Querkanals 115 geht in Richtung der Stutzenlängsachse 80 in die Förderleitung 30 über, die sich entlang der Stutzenlängsachse 80 vom konischen Teil 125 des Querkanals 115 bis zum Anschluß 35 erstreckt.

Im stromlosen Zustand des Elektromagneten 100 des Absperrventils 20 wird der tellerförmige Teil 107 von der Federkraft der Feder 104 an eine in den Querkanal 115 etwas hineinreichende Einbuchtung 110 am Gehäusedeckel 60 angedrückt und liegt an einer Innenwandung 111 der Einbuchtung 110 an. Im stromlosen Zustand ist das Absperrventil 20 offen, so daß aus der Umgebung Luft über den Strömungskanal 116 in den zylindrischen Teil 124 zum konischen Teil 125 des Querkanals 115 und von diesem über die Förderleitung 30 in den Adsorptionsfilter 6 strömen kann. Im in Figur 3 dargestellten bestromten Zustand des Elektromagneten 100 nimmt das Absperrventil 20 eine Schließstellung ein. Beim Bestromen des Elektromagneten 100 wird der tellerförmige Teil 107 von der Innenwandung 111 der Einbuchtung 110 zum Spulengehäuse 97 hin bewegt, um anliegend an einen Ventilsitz 112 den zylindrischen Teil 124 des Querkanal 115 vom konischen Teil 125 des Querkanals 115 abzudichten, so daß eine Strömungsverbindung vom Querkanal 115 zur Förderleitung 30 unterbrochen ist. Der zylindrische Teil 124 des Querkanals 115 ist mit einem etwas größeren Querschnitt als der konische Teil 125 des Querkanals 115 ausgebildet, so daß sich eine ringförmig umlaufende Ventilsitzfläche 113 ergibt, an der das Ventilschließglied 95 mit seinem tellerförmigen Teil 107 zum Beispiel mit einem elastischen Überzug dicht anliegen kann. Durch die teilweise konische Ausbildung 125 des Querkanals 115 ergibt sich der Vorteil, daß vom Adsorptionsfilter 6 über den Anschluß 35 in die Förderleitung 30 eindringendes Wasser oder Brennstoff nicht in den Bereich des Elektromagneten 100 des Absperrventils 20 gelangen kann, sondern aufgrund der konischen Ausgestaltung 125 des Querkanals 115 unter der Wirkung der Schwerkraft über den Strömungskanal 116 aus dem Gehäuse 36 abfließt.

Wie in Figuren 2 und 5 dargestellt ist, mündet der Strömungskanal 116 wie auch der Ansaugkanal 85 in eine an der unteren Begrenzungsfläche 38 am Gehäuse 36 vorgesehene Filteranformung 118. Die über die untere Begrenzungsfläche 38 sich erhebende Filteranformung 118 hat beispielsweise eine rechteckige Form und umschließt mehrere von der unteren Begrenzungsfläche 38 abstehende Noppen 119. Die Noppen 119 sind vorgesehen, um mit Abstand zur unteren Begrenzungsfläche 38 zwischen den Noppen 119 und einem nicht näher dargestellten, auf die Filteranformung 118 aufsetzbaren Filterdeckel den Umgebungsluftfilter 27 unterzubringen. Der in Figur 5 nicht näher dargestellte, beispielsweise rechteckförmige Umgebungsluftfilter 27 dient zum Reinigen der von der Pumpvorrichtung 2 angesaugten Umgebungsluft, die über den rechteckförmigen Strömungskanal 116 zum Absperrventil 20 und über den Ansaugkanal 85 zum Unterdruckventil 24 in den Förderraum 34 strömt.

Im folgenden wird die Arbeitsweise der Pumpvorrichtung 2 geschildert. Zu Beginn der Diagnose schließt das Regenerierventil 10 und das Absperrventil 20 wird bestromt, um in Schließstellung eine Strömungsverbindung von der Förderleitung 30 zur Umgebung zu unterbrechen. Anschließend oder gleichzeitig mit der Bestromung des Absperrventils 20 wird die Erregerspule 43 des Pumpteils 23 wechselweise von elektrischem Strom vorzugsweise mit relativ hoher Frequenz erregt und wieder entregt, so daß sich die Pumpmembran 22 in der Pumpausnehmung 70 hin- und herbewegt. Beim Bestromen der Erregerspule 43 des Pumpteils 23 wird der magnetische Anker 41 des Pumpteils 23 angezogen und bewegt sich mit der Pumpmembran 22 entgegen der Federkraft der Pumpfeder 39 zum Spulengehäuse 44 hin, wodurch sich bei geschlossenen Ventilen 24, 25 ein Unterdruck im Förderraum 34 einstellt. Bei einem bestimmten Unterdruck öffnet das Unterdruckventil 24, um Luft über den Ansaugkanal 85 aus der Umgebung in den Förderraum 34 einzusaugen. Im nachfolgenden stromlosen Zustand der Erregerspule 43 bewegt sich die Pumpmembran 22 aufgrund der Federkraft der Pumpfeder 39 in entgegengesetzter Richtung zum Gehäusedeckel 60 hin, wobei das Unterdruckventil 24 wieder schließt. Bei der Bewegung der Pumpmembran 22 zum Gehäusedeckel 60 hin, wird die im Förderraum 34 eingeschlossene Luft solange verdichtet, bis ein durch die Ausgestaltung des Überdruckventils 25 vorgebbarer Öffnungsüberdruck erreicht wird. Beim Erreichen des Öffnungsüberdrucks im Förderraum 34 öffnet das Überdruckventil 25, um die im Förderraum 34 verdichtete Luft über die Förderleitung 30 in den Adsorptionsfilter 6 abzugeben. Der Pumpvorgang mittels der Pumpmembran 22 ist nur eine zeitlang vorgesehen, bis sich nach einer bestimmten Anzahl von Pumpbewegungen der Pumpmembran 22 ein bestimmter Überdruck im Brennstoffverdunstungs-Rückhaltesystem 1 aufgebaut hat, wonach der elektromagnetische Antrieb beziehungsweise der Pumpteil 23 der Pumpmembran 22 abgeschaltet wird. Dies erfolgt dann, wenn die Federkraft der Pumpfeder 39 nicht mehr ausreicht die Pumpmembran 22 gegen die Druckkraft des Überdrucks im Förderraum 34 nach unten zu bewegen und damit mittels der Kontaktscheibe 67 Kontaktfahnen 120 zu schließen. Dadurch ergibt sich gegenüber einer Dichtheitsdiagnose mittels Unterdruck der Vorteil, daß ein ansonsten üblicher Drucksensor im Brennstoffverdunstungs-Rückhaltesystem 1 entfallen kann.

Ist keine Leckage im Brennstoffverdunstungs-Rückhaltesystem 1 vorhanden, so verbleibt die Pumpmembran 22 aufgrund des bei offenem Überdruckventil 25 dann ebenfalls im Förderraum 34 herrschenden Überdrucks gegen die Federkraft der Pumpfeder 39 an die Innenfläche 69 der Pumpausnehmung 70 gedrückt. Ist jedoch eine Leckage im Brennstoffverdunstungs-Rückhaltesystem 1 vorhanden, so baut sich der Überdruck ab, und die Pumpmembran 22 bewegt sich unterstützt durch die Federkraft der Pumpfeder 39 in Richtung des Gehäusedeckels 60, wobei die Kontaktscheibe 67 am Pumpstößel 40 eine in Figur 3 gestrichelt eingezeichnete Lage einnimmt.

In der gestrichelt eingezeichneten Lage der Kontaktscheibe 67 werden beispielsweise zwei elektrische Kontaktfahnen 120 elektrisch miteinander verbunden, mit deren Hilfe ein erneuter Pumpvorgang ausgelöst werden kann, und zwar solange, bis sich wieder ein bestimmter Überdruck im Brennstoffverdunstungs-Rückhaltesystem 1 aufgebaut hat. Dabei ist die Zeit, die zwischen der beispielsweise mehrmaligen Betätigung der Pumpvorrichtung 2 zur Wiederherstellung des Überdrucks benötigt wird, ein Maß für die Größe der aufgetretenen Leckageöffnung. Stellt sich jedoch selbst nach mehrmaligen Pumpvorgängen kein Überdruck ein, so kann dies auf eine besonders große Leckageöffnung oder auf einen fehlenden Tankdeckel am Brennstofftank 4 zurückgeführt werden.

Die Kontaktfahnen 120 sind im Bereich des Kontaktendes 51 des Pumpstößels 40 an einer in Figur 6 perspektivisch dargestellten Kontaktplatte 128 im Gehäuse 36 untergebracht. Wie in Figuren 3 und 4 dargestellt ist, ragen die beiden Kontaktfahnen 120 außerhalb des Spulengehäuses 44 des Pumpteils 23 in den von der Anformung 81 und dem eingesetzten Verschlußdeckel 68 begrenzten Raum. In der in Figur 3 gestrichelt dargestellten Lage der Kontaktscheibe 67 werden beide Kontaktfahnen 120 an einer dem Spulengehäuse 44 zugewandten Kontaktfläche 129 der Kontaktscheibe 67 miteinander verbunden, so daß eine elektrische Verbindung hergestellt wird. Die Kontaktscheibe 67 besitzt hierzu eine hochwertig bearbeitete Kontaktfläche 129, um eine Vielzahl elektrischer Kontakte, insbesondere ohne Funkenbildung, zu ermöglichen. Wie in der Figur 6 dargestellt ist, verfügt die Kontaktplatte 128 weiterhin über mehrere elektrische Bauteile 130, die zum Beispiel in Form von elektrischen Widerständen zur Ansteuerung des Pumpteils 23 und des Absperrventils 20 vorgesehen sind. Die elektrischen Bauteile 130 der Kontaktplatte 128 sind hierzu über abgewinkelte Kontaktbügel 131 und über in Figur 3 dargestellte Kontaktstifte 132 mit dem Elektromagneten 100 des Absperrventils 20 und dem Elektromagneten 46 des Pumpteils 23 elektrisch verbunden. Die Kontaktplatte 128 ist weiterhin über Steckerfahnen 133, die in einer an der oberen Begrenzungsfläche 37 am Gehäuse 36 angeformten Steckeraufnahme 134 eingebunden sind, von einem auf die Steckeraufnahme 134 aufsetzbaren elektrischen Stecker elektrisch kontaktierbar. Der Stecker stellt dabei eine Verbindung der Pumpvorrichtung 2 zu dem beispielsweise extern vorgesehenen elektronischen Steuergerät 21 her, das zum Beispiel zur getakteten Ansteuerung des Regenerierventils 10, zur Ansteuerung des Absperrventils 20 und des Pumpteils 23 sowie zur Auswertung der von der Pumpvorrichtung 2 abgegebenen elektrischen Signale vorgesehen ist. Mittels der elektrischen Bauteile 130 ist es möglich, wesentliche Ansteuerungsfunktionen der Pumpvorrichtung 2, insbesondere des Pumpteils 23 und des Absperrventils 20, selbsttätig, das heißt, ohne Zuhilfenahme des elektronischen Steuergeräts 21 vorzunehmen, so daß nur wenige Verbindungsleitungen zum elektronischen Steuergerät 21 erforderlich sind. Es ist jedoch auch möglich, eine entsprechende Auswerteschaltung im Gehäuse 36 selbst unterzubringen, die beispielsweise in Hybridbauweise ausgebildet und zum Beispiel auf der Kontaktplatte 128 aufgebracht ist.

## Patentansprüche

1. Pumpvorrichtung, insbesondere für ein Brennstoffverdunstungs-Rückhaltesystem einer Brennkraftmaschine, mit einer Pumpmembran, die einen Förderraum mit einer Ventilanordnung begrenzt, wobei durch ein erstes Ventil der Förderraum über eine Zuführleitung mit Umgebungsluft und durch ein zweites Ventil der Förderraum über eine Förderleitung mit einem Adsorptionsfilter verbindbar ist, und mit einem zwischen der Zuführleitung und der Förderleitung vorgesehenen Absperrventil, dadurch gekennzeichnet, daß die Pumpmembran (22) von einem elektromagnetischen Antrieb (23) bewegbar und das Absperrventil (20) elektromagnetisch betätigbar ausgebildet ist.

2. Pumpvorrichtung nach Anspruch 1, dadurch gekennzeichnet, daß die Pumpmembran (22) mit dem elektromagnetischen Antrieb (23)-und das elektromagnetisch betätigbare Absperrventil (20) in einem gemeinsamen Gehäuse (36) der Pumpvorrichtung (2) untergebracht sind.

3. Pumpvorrichtung nach Anspruch 1, dadurch gekennzeichnet, daß an der Pumpmembran (22) ein Pumpstößel (40) angebracht ist, der einen von einem Elektromagneten (46) bewegbaren magnetischen Anker (41) hat.

4. Pumpvorrichtung nach Anspruch 2, dadurch gekennzeichnet, daß das Absperrventil (20) ein Ventilschließglied (95) hat, an dem ein magnetischer Anker (96) angebracht ist, der von einem Elektromagneten (100) bewegbar ist.

5. Pumpvorrichtung nach Anspruch 4, dadurch gekennzeichnet, daß das Ventilschließglied (95) des Absperrventils (20) tellerförmig ausgebildet und in einem einen zylindrischen Querschnitt aufweisenden Teil (124) eines im Gehäuse (36) vorgesehenen Querkanals (115) untergebracht ist, der in einen einen konischen Querschnitt aufweisenden Teil (125) des Querkanals (115) übergeht, wobei an der Übergangsstelle des zylindrischen Teils (124) in den konischen Teil (125) ein Ventilsitz (112) des Ventilschließgliedes (95) ausgebildet ist.

6. Pumpvorrichtung nach Anspruch 2, dadurch gekennzeichnet, daß der elektromagnetische Antrieb (23) der Pumpmembran (22) und das Absperrventil (23) voneinander unabhängige Bewegungsachsen (64, 65) im Gehäuse (36) aufweisen.

7. Pumpvorrichtung nach Anspruch 2, dadurch gekennzeichnet, daß die Zuführleitung (29) von zwei im Gehäuse (36) separat vorgesehenen Kanälen (85, 116) gebildet wird.

8. Pumpvorrichtung nach Anspruch 2, dadurch gekennzeichnet, daß 'die Pumpmembran (22) in einer an einer Stirnfläche (62) des Gehäuses (36) vorgesehenen Ausnehmung (70, 71) angeordnet ist, die von einem auf die Stirnfläche (62) aufsetzbaren Gehäusedeckel (60) dicht verschließbar ist.

9. Pumpvorrichtung nach Anspruch 3, dadurch gekennzeichnet, daß an einem der Pumpmembran (22) gegenüberliegenden Ende (51) des Pumpstößels (40) eine die Lage des Pumpstößels (40) erfassende Einrichtung (67, 120) vorgesehen ist.

10. Pumpvorrichtung nach Anspruch 9, dadurch gekennzeichnet, daß die Lageerfassungseinrichtung (67, 120) von einer am Ende (51) des Pumpstößels (40) angebrachten Scheibe (67) und wenigstens zwei von der Scheibe (67) elektrisch miteinander verbindbaren Kontakten (120) gebildet wird.

11. Pumpvorrichtung nach Anspruch 2, dadurch gekennzeichnet, daß Mittel (128, 130) zur Ansteuerung des elektrischen Antriebs (23) der Pumpmembran (22) und des elektromagnetisch betätigbaren Absperrventils (20) im Gehäuse (36) oder am Gehäuse (36) vorgesehen sind.

12. Pumpvorrichtung nach Anspruch 10 und 11, dadurch gekennzeichnet, daß elektrische Bauteile (130) einer Kontaktplatte (128) mit dem Elektromagneten (46) des elektrischen Antriebs (23) und mit dem Elektromagneten (100) des Absperrventils (20) elektrisch verbunden sind und über die Kontaktfahnen (120) mittels der Scheibe (67) eine Ansteuerung des Elektromagneten (46) des elektrischen Antriebs (23) und des Elektromagneten (100) des Absperrventils (20) ermöglichen.

13. Pumpvorrichtung nach Anspruch 1, dadurch gekennzeichnet, daß zum Reinigen der von der Pumpvorrichtung (2) angesaugten Umgebungsluft ein Filter (27) vorgesehen ist.

## Claims

1. Pumping device, in particular for a fuel vapour control system of an internal-combustion engine, having a pumping diaphragm which bounds a delivery space with a valve arrangement, the delivery space being adapted such that, by means of a first valve, it can be connected to ambient air via a feed line and, by means of a second valve, it can be connected to an adsorption filter via a delivery line, and with a shut-off valve provided between the feed line and the delivery line, characterized in that the pumping diaphragm (22) is adapted such that it can be moved by an electromagnetic drive (23) and the shut-off valve (20) is designed such that it can be operated electromagnetically.

2. Pumping device according to Claim 1, characterized in that the pumping diaphragm (22) with the electromagnetic drive (23) and the electromagnetically operable shut-off valve (20) are accommodated in a common housing (36) of the pumping device (2).

3. Pumping device according to Claim 1, characterized in that fitted on the pumping diaphragm (22) is a pumping plunger (40), which has a magnetic armature (41) which can be moved by an electromagnet (46).

4. Pumping device according to claim 2, characterized in that the shut-off valve (20) has a valve closing element (95), fitted to which is a magnetic armature (96) which can be moved by an electromagnet (100).

5. Pumping device according to Claim 4, characterized in that the valve closing element (95) of the shut-off valve (20) is designed in the form of a plate and is accommodated in a cross-sectionally cylindrical part (124) of a transverse channel (115) provided in the housing (36) which merges with a cross-sectionally conical part (125) of the transverse channel (115), a valve seat (112) of the valve closing element (95) being formed at the transitional point of the cylindrical part (124) into the conical part (125).

6. Pumping device according to Claim 2, characterized in that the electromagnetic drive (23) of the pumping diaphragm (22) and the shut-off valve (23) have axes of movement (64, 65) which are independent of one another in the housing (36).

7. Pumping device according to Claim 2, characterized in that the feed line (29) is formed by two channels (85, 116) provided separately in the housing (36).

8. Pumping device according to Claim 2, characterized in that the pumping diaphragm (22) is arranged in a recess (70, 71) which is provided on an end face (62) of the housing (36) and can be closed in a sealed manner by a housing cover (60) which can be placed onto the end face (62).

9. Pumping device according to Claim 3, characterized in that a device (67, 120) sensing the position of the pumping plunger (40) is provided at an end (51) of the pumping plunger (40) lying opposite the pumping diaphragm (22).

10. Pumping device according to Claim 9, characterized in that the position-sensing device (67, 120) is formed by a disc (67) fitted on the end (51) of the pumping plunger (40) and at least two contacts (120) which can be electrically connected to one another by the disc (67).

11. Pumping device according to Claim 2, characterized in that means (128, 130) for activating the electric drive (23) of the pumping diaphragm (22) and of the electromagnetically operable shut-off valve (20) are provided in the housing (36) or on the housing (36) .

12. Pumping device according to Claims 10 and 11, characterized in that electrical components (130) of a contact plate (128) are electrically connected to the electromagnet (46) of the electric drive (23) and to the electromagnet (100) of the shut-off valve (20) and make an activation of the electromagnet (46) of the electric drive (23) and of the electromagnet (100) of the shut-off valve (20) possible via the contact lugs (120) by means of the disc (67).

13. Pumping device according to Claim 1, characterized in that a filter (27) is provided for cleaning the ambient air sucked in by the pumping device (2).

## Revendications

1. Dispositif de pompage, en particulier pour un système de retenue de vapeurs de carburant d'un moteur à combustion interne, comportant une membrane de pompe qui délimite avec un système de soupapes, une chambre de refoulement, une première soupape de cette chambre pouvant être reliée par une conduite d'amenée à l'air ambiant tandis qu'une deuxième soupape de la chambre peut être reliée par une conduite de refoulement à un filtre à adsorption, une soupape d'arrêt étant montée entre la conduite d'amenée et la conduite de refoulement,
caractérisé en ce que
la membrane de pompe (22) peut être déplacée par un entraînement électromagnétique (23) tandis que la soupape d'arrêt (20) peut être actionnée électromagnétiquement.

2. Dispositif de pompage selon la revendication 1,
caractérisé en ce que
la membrane de pompe (22) avec son entraînement électromagnétique (23) et la soupape d'arrêt (20) à actionnement électromagnétique sont montées dans un boîtier commun (36) du dispositif de pompage (2).

3. Dispositif de pompage selon la revendication 1,
caractérisé en ce que
sur la membrane de pompe (22) est montée une tige de pompe (40) équipée d'une armature (41) que peut déplacer un électroaimant (46).

4. Dispositif de pompage selon la revendication 2,
caractérisé en ce que
la soupape d'arrêt (20) comporte un obturateur de soupape (95) sur lequel est montée une armature magnétique (96) que peut déplacer un électroaimant (100).

5. Dispositif de pompage selon la revendication 4,
caractérisé en ce que
l'obturateur (95) de la soupape d'arrêt (20) a la forme d'un disque et est interposé dans une partie (124) à section cylindrique d'un canal transversal (115) prévu dans le boîtier (36), cette partie (124) débouchant dans une partie (125) à section conique du canal transversal (115), un siège de soupape (112) de l'obturateur (95) étant formé dans la zone de passage de la partie cylindrique (124) à la partie conique (125).

6. Dispositif de pompage selon la revendication 2,
caractérisé en ce que
l'entraînement électromagnétique (23) de la membrane de pompe (22) et la soupape d'arrêt (20) ont des axes de déplacement (64, 65) dans le boîtier (36) indépendants l'un de l'autre.

7. Dispositif de pompage selon la revendication 2,
caractérisé en ce que
la conduite d'amenée (29) est constituée de deux canaux (85, 116) prévus séparés dans le boîtier (36).

8. Dispositif de pompage selon la revendication 2,
caractérisé en ce que
la membrane de pompe (22) est logée dans un évidement (70, 71) creusé dans une face frontale (62) du boîtier (36) et qui peut être obturé de manière étanche par un couvercle de boîtier (60) emboîtable sur la face frontale (62).

9. Dispositif de pompage selon la revendication 3,
caractérisé en ce qu'
à l'extrémité (51) de la tige de pompe (40) opposée à la membrane de pompe (22) est prévu un dispositif (67, 120) détectant la position de la tige (40).

10. Dispositif de pompage selon la revendication 9,
caractérisé en ce que
le dispositif (67, 120) détecteur de position est constitué d'un disque (67) monté à l'extrémité (51) de la tige de pompe (40) et d'au moins deux contacts (120) qui peuvent être reliés électriquement par le disque (67).

11. Dispositif de pompage selon la revendication 2,
caractérisé en ce que
des moyens (128, 130) de commande de l'entraînement électrique (23) de la membrane de pompe (22) et de la soupape d'arrêt (20) à actionnement électromagnétique, sont prévus dans ou sur le boîtier (36).

12. Dispositif de pompage selon la revendication 10 ou 11,
caractérisé en ce que
des composants électriques (130) d'une plaque de contact (128) sont reliés électriquement à l'électroaimant (46) de l'entraînement électrique (23) et à l'électroaimant (100) de la soupape d'arrêt (20) et, à travers les languettes de contact (120) permettent par le disque (67) de commander l'électroaimant (46) de l'entraînement électrique (23) et l'électroaimant (100) de la soupape d'arrêt (20).

13. Dispositif de pompage selon la revendication 1,
caractérisé en ce qu'
un filtre (27) est prévu pour épurer l'air ambiant aspiré par le dispositif de pompage (2).
